Europäisches Patentamt
European Patent Office
Office européen des brevets

(19)

(11) Publication number: **0 273 987 A1**

(12)

# EUROPEAN PATENT APPLICATION

published in accordance with Art. 158(3) EPC

(21) Application number: 87904727.2

(22) Date of filing: 15.07.87

Data of the international application taken as a basis:

(86) International application number: PCT/JP87/00512

(87) International publication number: WO88/00585 (28.01.88 88/03)

(51) Int. Cl.³: C 07 D 215/22
C 07 D 491/113, A 61 K 31/47

(30) Priority: 15.07.86 JP 167394/86
15.07.86 JP 167395/86

(43) Date of publication of application: 13.07.88 Bulletin 88/28

(84) Designated Contracting States: AT DE

(71) Applicant: FUJI KAGAKU KOGYO KABUSHIKI KAISHA
55, Yokohoonji, Kamiichi-machi
Nakaniikawa-gun Toyama 930-05(JP)

(72) Inventor: IGARASHI, Kikuo
10, Nishino 7-chome
Itami-shi Hyogo 560(JP)

(72) Inventor: NAKAJIMA, Hiroshi
2256-27, Azaikeda, Tsujigado
Mizuhashi, Toyama-shi Toyama 939-05(JP)

(72) Inventor: KAWAI, Shinji
975, Hirono, Kamiichimachi
Nakaniikawa-gun Toyama 930-03(JP)

(72) Inventor: ENDO, Takeshi
975, Hirono, Kamiichimachi
Nakaniikawa-gun Toyama 930-03(JP)

(74) Representative: Vossius & Partner
Siebertstrasse 4 P.O. Box 86 07 67
D-8000 München 86(DE)

(54) **PROCESS FOR PREPARING TETRAHYDROQUINOLONE ETHERS AND NOVEL INTERMEDIATES THEREFOR.**

(57) A process for preparing a tetrahydroquinolone ether represented by formula (I) and a novel intermediate represented by formula (VIII) for preparing said ether; wherein $R^1$ represents a straight-chain or branched alkyl group having 1 to 4 carbon atoms and $R^3$ represents an alkylsulfonyloxy, arylsulfonyloxy, acyloxy or hydroxy group or a halogen atom. This process is characterized in that a 3-substituted 1,2-propylene oxide or 3-substituted 1,2-propanediol is reacted with octahydroquinoline-2,5-dione in the presence of an acid catalyst to produce a compound of the above general formula (VII), which is then reacted with an alkylamine represented by the formula of $R^1$-$NH_2$ (wherein $R^1$ is as defined above) to produce a novel compound represented by formula (VIII), (wherein $R^1$ is as defined above), which is then reacted with a dehydrogenative ring opening reagent to obtain the compound of formula (I).

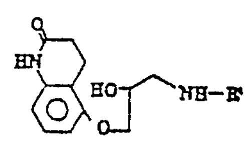
(I)

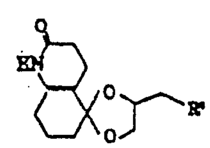
(VII)

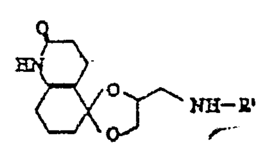
(VIII)

BAD ORIGINAL

EP 0 273 987 A1

0273987

SPECIFICATION

PROCESS FOR PREPARING TETRAHYDROQUINOLONE ETHERS, AND NEW INTERMEDIATES THEREFOR

Background of the Invention

The present invention relates to a new process for the preparation of tetrahydroquinolone ethers of the general formula:

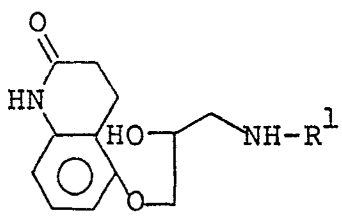

(I)

wherein $R^1$ is a straight or branched chain alkyl group having 1 to 4 carbon atoms, or acid addition salts thereof.

Tetrahydroquinolone ether hydrochloride of the formula:

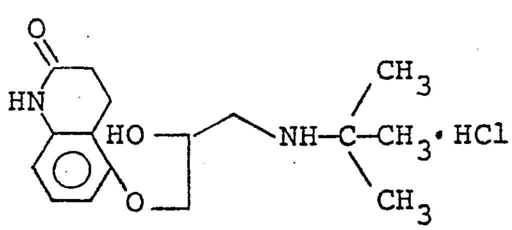

(II)

generically termed carteolol, is a valuable substance in practical use as a β-blocker, in particular as a therapeutic principle for essential hypertension, angina pectoris,

cardiac neurosis, arrhythmia and the like disorders.

The present invention provides a new industrial process for preparing tetrahydroquinolone ethers of the general formula (I) including carteolol, i.e. 5-(3-tert.-butylamino-2-hydroxy)propoxy-3,4-dihydrocarbostyril of the formula (II) above.

Background Art

For preparing 3,4-dihydrocarbostyril compounds of the general formula

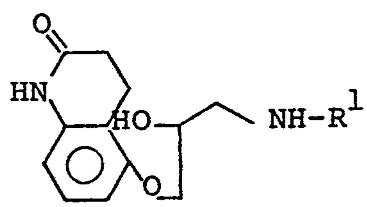

(I)

wherein $R^1$ is a straight or branched chain alkyl group having 1 to 4 carbon atoms, a wide variety of processes have been hitherto used, wherein the compound 5-hydroxycarbostyril or 5-hydroxy-3,4-dihydrocarbostyril is used as starting material and a 3-alkylamino-2-hydroxypropyl group introduced into the 5-hydroxyl group to give an ether-type compound. For example, in processes disclosed in U.S. Patent Nos. 3,910,924, 4,256,890 and 4,329,468, the compounds of the general formula (I) are prepared by reacting 5-hydroxy-3,4-dihydrocarbostyril with an epihalogenohydrin or 3-halogeno-1,2-propanediol and then reacting the resultant 5-(2,3-epoxy)propoxy- or 5-(2,3-dihydroxy)propoxy-3,4-dihydrocarbostyril with an alkylamine.

In an alternative process as described in Japanese Patent Publication No. 19584/1978, the 3,4-dihydrocarbostyril compounds of the general formula (I) are obtained by reacting the above mentioned 5-(2,3-epoxy)propoxy- or 5-(2,3-dihydroxy)propoxy-3,4-dihydrocarbostyril with a benzylalkylamine of the general formula:

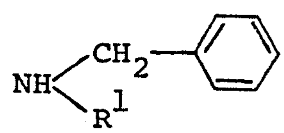

wherein $R^1$ has the same meaning as given above, and reducing the resultant (3-benzylalkylamino-2-hydroxy)propoxy-3,4-dihydrocarbostyril compound to split off the benzyl group.

These prior art processes, however, have yet to be improved in many respects, if they are to be industrially applied, since they do not achieve satisfactory overall yields and the starting 5-hydroxy-3,4-dihydrocarbostyril is expensive.

## Disclosure of the Invention

The present inventors have now succeeded in establishing an industrial process for preparing in efficient manner tetrahydroquinolone ethers of the general formula (I) shown

above from 1, 2, 3, 4, 5, 6, 7, 8-octahydroquinoline-2,5-dione of the formula (III):

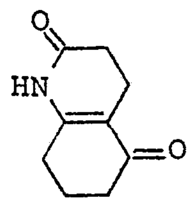

(III)

which is a readily available known compound and inexpensive material, by way of an octahydro-spiro[1,3-dioxolan -2,5'-quinoline-2'-one] of the general formula (IV):

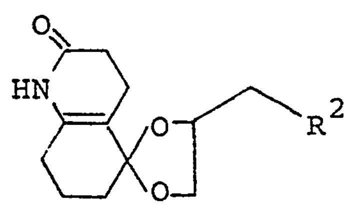

(IV)

wherein $R^2$ stands for alkylamino, alkylsulphonyloxy, arylsulphonyloxy, acyloxy, hydroxy or halogen. The term "alkyl" as used herein means straight or branched chain alkyl having 1 to 4 carbon atoms.

The compounds of the general formula (IV) above are new compounds created by the inventors which have not been described in the literature.

The present invention thus provides a process for preparing tetrahydroquinolone ethers of the general formula (I) above in steps (1) and (2) mentioned below. Furthermore

the present invention provides new compounds of the general formula (IV) above useful as intermediates for such new process.

The new process of the invention mentioned above comprises the following steps:

(1) Octahydroquinoline-2,5-dione of the formula (III) shown above is reacted in the presence of an acid catalyst with a 3-substituted-1,2-propylene oxide of the general formula (V):

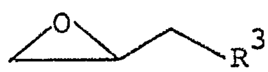

(V)

wherein $R^3$ represents p-$CH_3C_6H_4SO_2O$- , $CH_3SO_2O$- , $C_6H_5COO$- , OH or halogen, or a 3-substituted-1,2-propanediol of the general formula (VI):

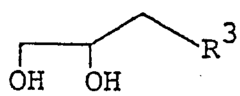

(VI)

wherein $R^3$ has the same meaning as given above, to give a 1',2',3',4',5',6',7',8'-octahydro-4-substituted methyl-spiro[1,3-dioxolan-2,5'-quinoline-2'-one] of the general formula (VII):

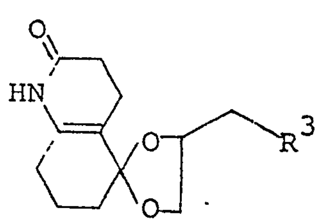

(VII)

wherein $R^3$ has the same meaning as given above.

(2) The resultant spiro [1,3-dioxolan—2,5'-quinoline-2'-one] of the general formula (VII) above is reacted with an alkylamine of the formula $R^1-NH_2$ wherein $R^1$ has the same meaning as given above to convert the group $R^3$ into an alkylamino group ($R^1$-NH-), and the thus obtained spiro compound of the general formula (VIII):

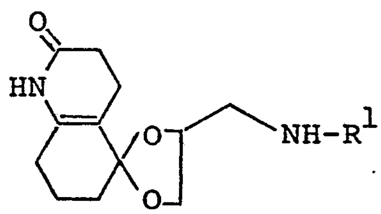

(VIII)

wherein $R^1$ has the same meaning as given above, is reacted with a dehydrogenating / ring cleaving reagent to give a tetrahydroquinolone ether of the general formula (II) shown above.

The steps (1) and (2) in the process of the present invention are schematically shown in the following, wherein each symbol in the formulas has the same meaning as given above.

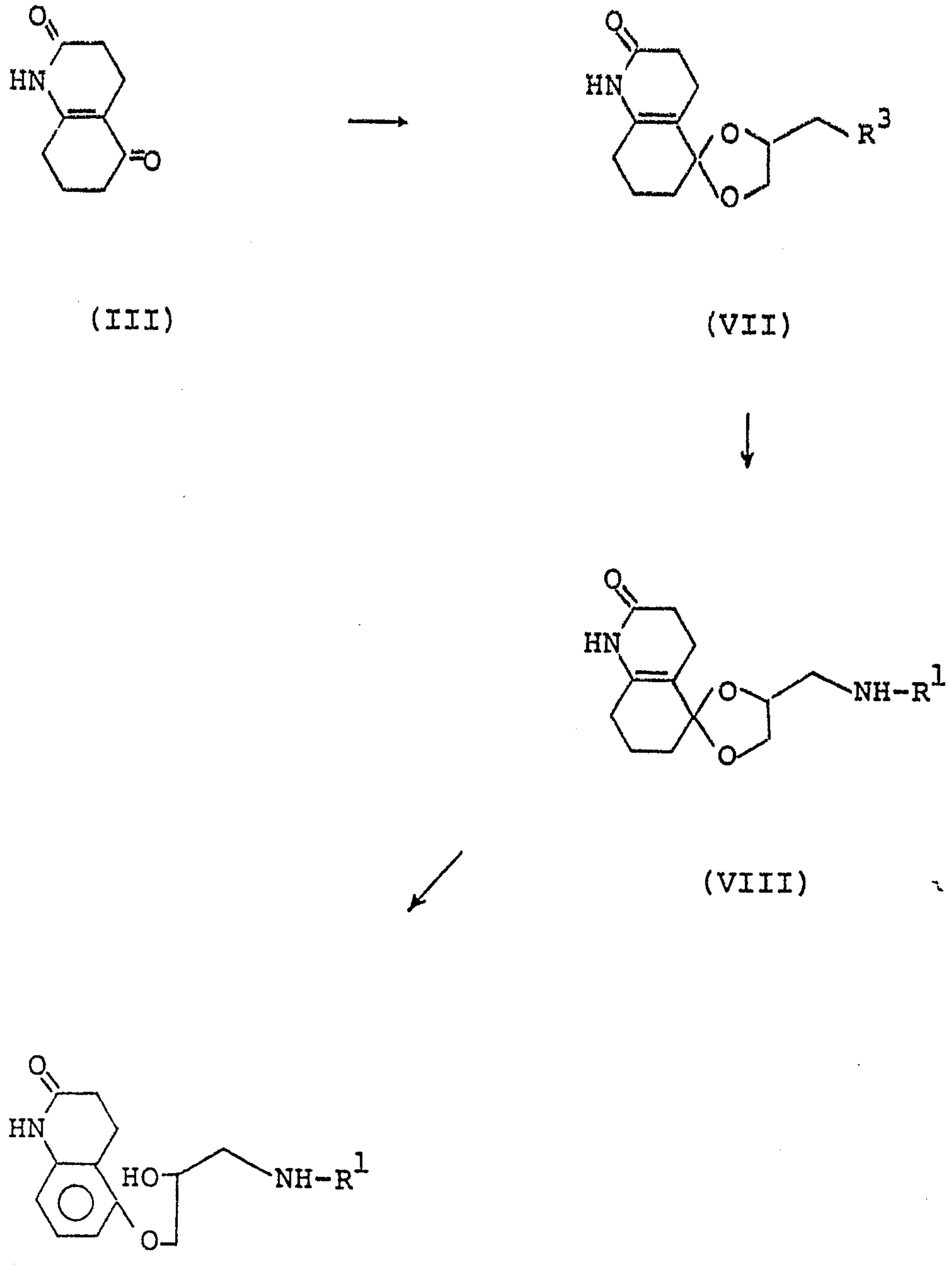

(III)

(VII)

(VIII)

(I)

Each of the steps mentioned above will now be described in more detail in the following.

The starting material used in the new process of the present invention, i.e. 1, 2, 3, 4, 5, 6, 7, 8-octahydroquinoline-2,5-dione, can be synthesized in simple and inexpensive manner known from the literature (Tetrahedron Letters, pp. 2441-2444, 1965). To this compound is added, as described above, a 3-substituted-1,2-propylene oxide (V). Examples of solvents used in the addition reaction include alkyl halides such as methylene chloride, chloroform, carbon tetrachloride and dichloroethane as well as those conventionally used in acid-catalyzed reactions such as ethers, toluene, xylene and nitrobenzene. There is used an acid catalyst in a proportion of from 0.05 to 0.5 mole per mole of the dione compound (III) mentioned above. Illustrative of such acid catalysts are anhydrous stannic chloride, ferric chloride, titanium tetrachloride and aluminum chloride. The reaction temperature may range from such temperature as to give chilly conditions to room temperature.

In carrying out the dehydration / condensation between the dione compound (III) and a 3-substituted-1,2-propanediol (VI), it is preferred to use sulphuric acid or p-toluenesulphonic acid as the acid catalyst and a somewhat elevated temperature as the reaction temperature. It is preferred to

use a solvent so selected that a preferred temperature range of from 70 to 120°C may be maintained with heating under reflux. Illustrative of such solvents are benzene, toluene, xylene, tetrahydrofuran and alkyl halides. Since the reaction is dehydration it is naturally preferred to dispel the water formed out of the reaction system. It is therefore most preferred to use such solvent as will easily form an azeotropic mixture with water.

The 3-substituted-1,2-propylene oxide (V) or 3-substituted-1,2-propanediol (VI) is used in a proportion of from 1 to 10 moles per mole of the dione compound (III), and the organic solvent in an amount five to fifty times the weight of the dione compound (III).

The most economically preferred embodiment of the substituent $R^3$ in the 3-substituted-1,2-propylene oxides of the formula (V) and the 3-substituted-1,2-propanediols of the formula (VI) is a chlorine atom.

The above mentioned 4-alkylaminomethyl-spiro compounds of the formula (VIII) can be obtained by subjecting a 4-substituted methyl-spiro compound of the formula (VII) to a nucleophilic substitution reaction with an alkylamine of the formula $R^1$—$NH_2$ in the presence of an alkali iodide, copper iodide or tetraethylammonium halide. Thus, the spiro compound (VII) is dissolved in an organic solvent such as isopropyl alcohol, dimethyl sulphoxide, toluene or xylene. To this

spiro compound (VII) are added 0.05 to 1 equivalent of the alkali iodide, copper iodide or tetraethylammonium halide and the alkylamine in an amount three to ten times the weight of the spiro compound (VII), and the reaction is effected for 5 to 24 hours while heating under reflux. The amount of the solvent used in this reaction is preferably ten to thirty times the weight of the spiro compound (VII). In order to accelerate the reaction, it is also possible to effect it in a sealed tube at a relatively high temperature to make the reaction complete within a shorter period of time.

In an embodiment to carry out the ring cleavage of 4-substituted methyl-spiro[1,3-dioxolan—2,5'-quinoline-2'-ones] of the formula (VII) or (VIII) above, one such compound is dissolved in such a solvent as a halogenated hydrocarbon. With ice cooling or at room temperature for the first 0.5 to 2 hours a dehydrogenating / ring-cleaving reagent as will be mentioned below is,when it is in liquid form, slowly added dropwise as such, or when it is in crystalline form, added as such or dropwise in solution. A period of 8 to 12 hours is allowed at room temperature for ripening. There are thus easily obtained tetrahydroquinolone ethers of the formula (I).

Examples of solvents usable in this reaction include low-boiling halogenated hydrocarbons such as methylene chloride, chloroform and dichloroethane as well as diethyl ether, ethyl acetate, benzene, toluene and the like.

Usually, the reaction temperature is preferably selected between chilly temperature and room temperature, and when palladium-carbon is used as the reagent a temperature between 100 and 200°C is selected. It usually suffices to initiate the dehydrogenation/ketal ring-cleavage reaction with ice cooling, while heating if necessary, and no special equipment is required. Preferred examples of the dehydrogenating/ketal ring-cleaving reagent include 2,3-dichloro-5,6-dicyanobenzoquinone, palladium-carbon, phenyltrimethylammonium perbromide, pyridinium hydrobromide perbromide and bromine.

The tetrahydroquinolone ethers of the formula (I) obtained by the process of the invention can be converted into corresponding acid addition salts in conventional manner by reacting such tetrahydroquinolone ethers with desired acids dissolved in alcohols.

The advantages characteristic of the process of the invention consist in that the end product can be obtained in high yield and also in that by-products, theoretically possible to form, are not produced at all. In addition thereto, no unusual conditions are required and operation is extremely straightforward and simple in carrying out the process of the present invention.

The present invention will now be illustrated in more detail by way of the following illustrative examples.

Example 1

3.00 Grams of 1,2,3,4,5,6,7,8-octahydroquinoline-2,5-dione (of the formula III) and 7.46 g of epibromohydrin are added to 30 ml of methylene chloride and the mixture is stirred at 0°C on an ice bath to give a solution. To this solution is added 1.05 ml of stannic chloride, and the mixture is further stirred for 2.5 hours. After addition of 15.00 g of anhydrous potassium carbonate stirring is applied for a further 30 minutes. The reaction liquid is filtered to remove insolubles and the filtrate is added dropwise to 150 ml of a 10% aqueous solution of potassium carbonate. 150 Millilitres of methylene chloride is added and the mixture is shaken. The organic layer is washed with 100 ml of a saturated aqueous solution of sodium chloride and dried over anhydrous sodium sulphate. The drying agent is separated, the filtrate is concentrated under reduced pressure and the crystalline residue is washed with 15 ml of isopropyl alcohol to give 4.50 g of 4-bromomethyl-1',2',3',4',5',6',7',8'-octahydro-spiro[1,3-dioxolan—2,5'-quinoline-2'-one] (of the formula VII wherein $R^3$=Br) (yield: 82.0%).
M.p. 186-187°C (methanol), colorless prisms.

$^1$H-NMR : δ(ppm) 1.6-2.7 (m, 10H), 3.2-4.6 (m, 5H), 8.29 (bs, 1H), ($CDCl_3$).

$^{13}$C-NMR : δ(ppm) 18.37, 18.58, 19.40, 19.61, 26.33, 26.44, 30.67, 32.35, 32.51, 33.05, 35.00, 68.65, 69.41, 74.44, 76.18, 108.90, 109.66, 109.82, 110.10, 135.78, 136.48, 171.86 ($CDCl_3$).

IR : ($cm^{-1}$), 1650.

Elementary analysis : as $C_{12}H_{16}BrNO_3$

| | C | H | N |
|---|---|---|---|
| Calcd. (%) | 47.70 | 5.34 | 4.64 |
| Found (%) | 47.78 | 5.52 | 4.74 |

Example 2

1.50 Grams of the product (of the formula VII wherein $R^3$ = Br ) obtained in Example 1, 12.00 g of t-butylamine and 0.25 g of sodium iodide are added to 10 ml of isopropyl alcohol and the mixture is heated under reflux at 80-90°C for 11 hours. The reaction liquid is then concentrated under reduced pressure and 100 ml of chloroform and 100 ml of a 10% aqueous solution of potassium carbonate are added to the residue. The mixture is then shaken and the organic layer is washed with 75 ml of a saturated aqueous solution of sodium chloride and dried over anhydrous sodium sulphate. The drying agent is separated and the filtrate is distilled under reduced pressure to obtain 1.35 g of 4-t-butylaminomethyl-1',2',3',4',5',6',7',8'-octahydro-spiro[1,3-dioxolan-2,5'-quinoline-2'-one]. (Yield: 92.4%)

M.p. 160°C (ethyl acetate), colorless prisms.

$^1$H-NMR : δ(ppm) 1.10 (s, 9H), 1.4-2.6 (m), 2.70 (d, 2H, 9.5Hz), 3.66 (t, 2H), 4.0-4.4 (m, 3H), 8.02 (bs, 1H), ($CDCl_3$).

$^{13}$C-NMR : δ (ppm) 18.64, 19.72, 26.44, 29.04, 33.07, 35.27,

45.95, 50.17, 68.32, 77.96, 108.52, 110.80, 135.18, 170.75 ($CDCl_3$).

IR : ($cm^{-1}$), 1660.

Elementary analysis : as $C_{16}H_{26}N_2O_3$

| | C | H | N |
|---|---|---|---|
| Calcd. (%) | 65.28 | 8.90 | 9.52 |
| Found (%) | 65.45 | 8.96 | 9.51 |

Example 3

1.00 Gram of the 2,5-dione compound (III) and 1.68 g of epichlorohydrin are added with stirring to 10 ml of methylene chloride and dissolved therein. To this solution is added 0.7 ml of stannic chloride at room temperature and the mixture is stirred for one hour. 8.00 Grams of anhydrous potassium carbonate is added and stirring is continued for a further 30 minutes. The reaction liquid is added dropwise to 100 ml of water and 90 ml of methylene chloride is added thereto. The mixture is shaken and phase separated. The organic layer is washed with 100 ml of a saturated aqueous solution of sodium chloride and dried over anhydrous sodium sulphate. The liquid resulting upon separation of the drying agent is distilled under reduced pressure to remove the solvent. The residue is washed with isopropyl alcohol to obtain 1.40 g of 4-chloromethyl-1',2',3',4',5',6',7',8'-octahydro-spiro[1,3-dioxolan -2,5'-quinoline-2'-one] (of the

formula VII wherein $R^3$ = Cl ) (Yield: 89.7%)

M.p. 174-175°C (methanol), colorless prisms.

$^1$H-NMR : δ(ppm) 1.6-2.7 (m, 10H), 3.3-4.6 (m, 5H), 8.42 (bs, 1H) ($CDCl_3$).

$^{13}$C-NMR : δ(ppm) 18.31, 18.58, 19.40, 19.61, 26.39, 30.67, 32.94, 34.90, 44.48, 67.73, 68.43, 74.60, 76.45, 108.90, 109.50, 109.66, 110.04, 135.78, 136.54, 171.91 ($CDCl_3$).

IR : ($cm^{-1}$), 1650.

Elementary analysis : as $C_{12}H_{16}ClNO_3$

| | C | H | N |
|---|---|---|---|
| Calcd. (%) | 55.93 | 6.26 | 5.44 |
| Found (%) | 56.17 | 6.41 | 5.74 |

Example 4

4.51 Grams of the product (of the formula VII wherein $R^3$ = Cl) obtained in Example 3, 45.00 g of t-butylamine and 0.70 g of sodium iodide are added to 50 ml of isopropyl alcohol and the mixture is heated in a sealed tube at 80-90°C for 28 hours. The reaction liquid is then distilled under reduced pressure and 500 ml of chloroform and 400 ml of a saturated aqueous solution of sodium hydrogen carbonate are added to the residue. The mixture is shaken, the organic layer is dried over anhydrous sodium sulphate, and the drying agent is separated. The filtrate is distilled under reduced pressure. From the residue is obtained 3.03 g of 4-t-butylaminomethyl-spiro product identical with the product obtained in Example 2 (yield : 58.8%).

Example 5

20.00 Grams of the same starting 2,5-dione compound as used in Example 1, 66.90 g of 3-chloro-1,2-propanediol and 2.30 g of p-toluenesulphonic acid hydrate are added to 300 ml of toluene and the mixture is heated under reflux for 24 hours. The water formed during this period is fractionated and discarded. The reaction liquid is distilled under reduced pressure, and 300 ml of chloroform and 200 ml of a 2% aqueous solution of sodium carbonate are added to the residue. The mixture is shaken and the organic layer is washed with 200 ml of water and dried over anhydrous sodium sulphate. The drying agent is separated and the filtrate is distilled under reduced pressure. The residue is recrystallized from methanol to obtain 15.60 g of 4-chloromethyl-spiro product identical with the product obtained in Example 3 (yield: 50.0%).

Example 6

7.06 Grams of the same starting 2,5-dione compound as used in Example 1 and 15.3 g of methanesulphonyloxymethyl-oxysilane are added to 300 ml of methylene chloride with stirring and dissolved therein. To this solution is added

4.5 ml of stannic chloride at room temperature and the mixture is stirred for one hour. After addition of 50 g of anhydrous potassium carbonate, the mixture is stirred for a further 30 minutes. The reaction liquid is filtered and the filtrate is added dropwise to 500 ml of a 10% aqueous solution of potassium carbonate. After 500 ml of methylene chloride is added, the mixture is shaken and phase separated. The organic layer is washed with 400 ml of a saturated aqueous solution of sodium chloride, then with 400 ml of water, and dried over anhydrous sodium sulphate. The reaction liquid resulting upon separation of the drying agent is distilled under reduced pressure to remove the solvent. The residue is recrystallized from isopropanol with a small amount of triethylamine added to obtain 8.72 g of 4-methanesulphonyl-oxymethyl-1',2',3',4',5',6',7',8'-octahydro-spiro[1,3-dioxolan—2,5'-quinoline-2'-one] (of the formula VII wherein $R^3$ = $CH_3-SO_2O$) (yield: 64.3%).

M.p. 130-133°C (isopropanol), colorless prisms.

$^1$H-NMR : δ(ppm) 1.5-2.7 (m, 10H), 3.07 (s, 3H), 3.5-3.95 (m, 1H), 4.0-4.6 (m, 4H), 7.43 (bs, 1H) ($CDCl_3$)

IR : ($cm^{-1}$), 1680.

Elementary analysis : as $C_{13}H_{19}NO_6S$

| | C | H | N |
|---|---|---|---|
| Calcd. (%) | 49.20 | 6.03 | 4.41 |
| Found (%) | 49.28 | 6.21 | 4.39 |

Example 7

1.21 Grams of 4-bromomethyl-1',2',3',4',5',6',7',8'-octahydro-spiro[1,3-dioxolan—2,5'-quinoline-2'-one] obtained in Example 1 and 4.92 g of tetraethylammonium acetate are added to 30 ml of dimethylformamide and the mixture is heated with stirring on a water bath at 80-90°C for 17 hours. This solution is distilled under reduced pressure to remove the solvent. To the residue are added 70 ml of water and 100 ml of chloroform and the mixture is shaken. The organic layer is washed with 70 ml of water and dried over anhydrous sodium sulphate. The reaction liquid resulting upon separation of the drying agent is distilled under reduced pressure to remove the solvent. The residue is recrystallized from isopropanol to obtain 0.82 g of 4-acetyloxymethyl-1',2',3',4',5',6',7',8'-octahydro-spiro[1,3-dioxolan—2,5'-quinoline-2'-one] (of the formula VII wherein $R^3$ = $OOCCH_3$ ) (yield: 72.8%).

M.p. 144°C (isopropanol).

$^1$H-NMR : δ(ppm) 1.5-2.5 (m, 13H), 2.06 (s, 3H), 3.6-3.9 (m, 1H), 4.0-4.6 (m, 4H) ($CD_3OD$).

$^{13}$C-NMR : δ(ppm) 19.34, 19.56, 20.53, 20.70, 27.04, 31.59, 33.97, 35.75, 65.51, 67.51, 74.34, 76.51, 110.3[illegible], 110.42, 110.64, 111.67, 137.24, 137.89, 172.45, 173.70 ($CD_3OD$).

IR : ($cm^{-1}$), 1740, 1660.

Elementary analysis : as $C_{14}H_{19}NO_5$

| | C | H | N |
|---|---|---|---|
| Calcd. (%) | 59.78 | 6.81 | 4.98 |
| Found (%) | 59.90 | 6.98 | 4.96 |

Example 8

1.53 Grams of 4-acetyloxymethyl-1',2',3',4',5',6',7',8'-octahydro-spiro[1,3-dioxolan—2,5'-quinoline-2'-one] obtained in Example 7 is added with stirring to 100 ml of methanol and dissolved therein. To this solution is added 60 mg of a 72% solution of sodium methoxide in methanol and the mixture is allowed to stand overnight at room temperature. The solvent is distilled off under reduced pressure and the residue is recrystallized from methanol-ethyl acetate to obtain 0.99 g of 4-hydroxymethyl-1',2',3',4',5',6',7',8'-octahydro-spiro[1,3-dioxolan—2,5'-quinoline-2'-one] (of the formula VII wherein $R^3$ = OH ) (yield: 76.1%).

M.p. 138-141°C/methanol-ethyl acetate), colorless prisms.

$^1$H-NMR: δ(ppm) 1.6-2.5 (m, 10H), 3.5-3.9 (m, 3H), 3.95-4.4 (m, 2H) ($CD_3OD$).

$^{13}$C-NMR: δ(ppm) 19.29, 19.56, 20.53, 20.70, 26.98, 31.53, 34.08, 35.87, 63.72, 67.78, 77.01, 79.43, 109.88, 111.07, 112.05, 136.91, 137.51, 173.75 ($CD_3OD$).

IR : ($cm^{-1}$), 1655.

Elementary analysis : as $C_{12}H_{17}NO_4$

| | C | H | N |
|---|---|---|---|
| Calcd. (%) | 60.23 | 7.16 | 5.85 |
| Found (%) | 60.41 | 7.29 | 5.90 |

Example 9

4.98 Grams of 4-t-butylaminomethyl-1',2',3',4',5',6',7',8'-octahydro-spiro[1,3-dioxolan-2,5'-quinoline-2'-one] obtained in Example 2 is dissolved in 50 ml of methylene chloride. To this solution is added dropwise a solution of 2.70 g of bromine in 20 ml of methylene chloride, and the mixture is stirred for 2 hours at room temperature. To the reaction liquid are then added 100 ml of methylene chloride and 150 ml of a 10% aqueous solution of potassium carbonate, and the mixture is stirred for 5 minutes and phase separated. The organic layer is washed with 200 ml of a saturated aqueous solution of sodium chloride and dried over anhydrous sodium sulphate. The drying agent is filtered off and the filtrate is distilled under reduced pressure to remove the solvents. To the free base residue is added 100 ml of a 20% solution of hydrogen chloride in methanol to dissolve the same. The solvent is distilled off under reduced pressure, and the residue is crystallized from methanol to obtain 4.02 g of 5-(3-t-butylamino-2-hydroxy)propyloxy-1,2,3,4-tetrahydro-quinoline-2-one hydrochloride (yield: 72.3%).

M.p. 274-275°C (decomp.) (methanol-water).

Elementary analysis : as $C_{16}H_{24}N_2O_3 \cdot HCl$

| | C | H | N |
|---|---|---|---|
| Calcd. (%) | 58.44 | 7.66 | 8.52 |
| Found (%) | 58.64 | 7.77 | 8.53 |

This reaction product was completely identical in different analytical results with well known carteolol as commercially available product.

Example 10

2.34 Grams of the same starting ketal compound as used in Example 9 is dissolved in 50 ml of methylene chloride and 2.11 g of pyridinium hydrobromide perbromide is added thereto. The mixture is stirred overnight at room temperature to effect reaction. To the reaction liquid is then added 100 ml of methylene chloride and 100 ml of a 10% aqueous solution of potassium carbonate and the mixture is shaken and phase separated. The organic layer is washed with 200 ml of a saturated aqueous solution of sodium chloride and dried over anhydrous magnesium sulphate. The drying agent is filtered off, and the solvents are distilled off from the filtrate under reduced pressure. To the residue is added 100 ml of a 20% solution of hydrogen chloride in methanol to dissolve the same, and the solvent is distilled off under reduced

pressure. The resulting residue is worked up as described in Example 9 to obtain 2.05 g of carteolol hydrochloride (yield: 78.4%).

Example 11

3.75 Grams of the same starting ketal compound as used in Example 9 is dissolved in 50 ml of methylene chloride,and 4.42 g of phenyltrimethylammonium perbromide is added thereto. The mixture is stirred for 2.5 hours at room temperature. To the reaction liquid are then added 100 ml of chloroform and 150 ml of a 10% aqueous solution of potassium carbonate. The mixture is stirred for 5 minutes and then subjected to phase separation. The organic layer is washed with 200 ml of a saturated aqueous solution of sodium chloride and dried over anhydrous sodium sulphate. The drying agent is filtered off and the solvents are distilled off from the filtrate under reduced pressure. To the residue is added 100 ml of a 20% solution of hydrogen chloride in methanol to dissolve the same and the solvent and the like are distilled off under reduced pressure. The resulting residue is worked up as described in Example 9 to obtain 2.39 g of carteolol hydrochloride (yield: 57.0%).

Example 12

2.64 Grams of the same starting 4-t-butylaminomethyl-

spiro compound as used in Example 9 is dissolved in 50 ml of toluene, and 1.61 g of trifluoromethanesulphonic acid is added thereto. After addition of 2.03 g of 2,3-dichloro-5,6-dicyano-1,4-benzoquinone, the mixture is stirred for 45 minutes at room temperature. To the reaction liquid are added 300 ml of chloroform and 150 ml of a 10% aqueous solution of sodium hydroxide and the mixture is shaken vigorously. The same work-up procedure as described in Example 9 is then followed to obtain carteolol hydrochloride.

C L A I M S

1. A process for the preparation of tetrahydroquinolone ethers of the general formula:

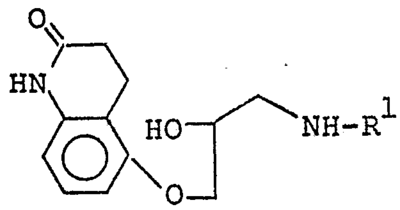

(I)

wherein $R^1$ stands for a straight or branched chain alkyl group having 1 to 4 carbon atoms, or acid addition salts thereof, which comprises reacting octahydroquinoline-2,5-dione of the formula:

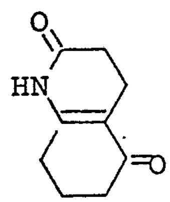

(III)

in the presence of an acid catalyst with a 3-substituted-1,2-propylene oxide or a 3-substituted-1,2-propanediol to give an octahydro-4-substituted methyl-spiro[1,3-dioxolan-2,5'-quinoline-2'-one] of the general formula:

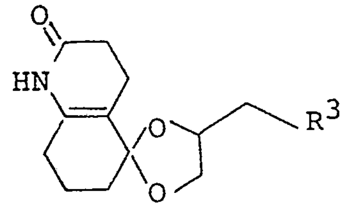

(VII)

wherein $R^3$ stands for alkylsulphonyloxy, arylsulphonyloxy, acyloxy, hydroxy or halogen, reacting the compound of the formula (VII) with an alkylamine of the general formula:

$$R^1-NH_2$$

wherein $R^1$ has the same meaning as given above, to give a 4-alkylaminomethyl-spiro[1,3-dioxolan -2,5'-quinoline-2'-one] compound of the general formula:

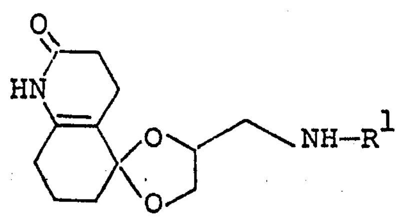

(VIII)

wherein $R^1$ has the same meaning as given above, and then reacting the compound (VIII) with a dehydrogenating / ring cleaving reagent.

2. A process according to claim 1, wherein the acid catalyst is stannic halide, ferric halide, titanium halide, aluminum halide, sulphuric acid or p-toluenesulphonic acid.

3. A process according to claim 1, wherein the dehydrogenating / ring cleaving reagent is selected from 2,3-dichloro-5,6-dicyanobenzoquinone, palladium-carbon, phenyltrimethylammonium perbromide, pyridinium hydrobromide perbromide or bromine.

4. A process according to claim 1, wherein the 3-substituted-1,2-propylene oxide is selected from epichloro-

hydrin, epibromohydrin and methanesulphonyloxymethyloxysilane.

5. A process according to claim 1, wherein the $R^1$ is a t-butylamino group.

6. 1',2',3',4',5',6',7',8'-Octahydro-4-substituted methyl-spiro[1,3-dioxolan -2,5'-quinoline-2'-ones] of the general formula (IV);

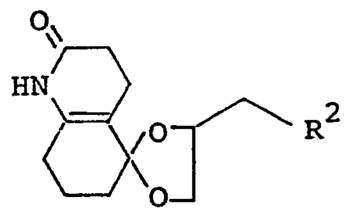

(IV)

wherein $R^2$ stands for alkylamino, alkylsulphonyloxy, arylsulphonyloxy, acyloxy, hydroxy or halogen, as intermediates for the preparation of said tetrahydroquinolone ethers of the general formula (I).

7. The spiro compound according to claim 6, wherein the $R^2$ is a t-butylamino group.

8. The spiro compound according to claim 6, wherein the $R^2$ is a tosyloxy group.

9. The compound according to claim 6, wherein the $R^2$ is a mesyloxy group.

10. The spiro compound according to claim 6, wherein the $R^2$ is a chlorine atom.

11. The spiro compound according to claim 6, wherein the $R^2$ is a bromine atom.

# INTERNATIONAL SEARCH REPORT

International Application No PCT/JP87/00512

**I. CLASSIFICATION OF SUBJECT MATTER** (if several classification symbols apply, indicate all)

According to International Patent Classification (IPC) or to both National Classification and IPC

$Int.Cl^4$ C07D215/22, C07D491/113, A61K31/47

**II. FIELDS SEARCHED**

Minimum Documentation Searched

| Classification System | Classification Symbols |
|---|---|
| IPC | C07D215/22, C07D491/113, A61K31/47 |

Documentation Searched other than Minimum Documentation to the Extent that such Documents are Included in the Fields Searched

**III. DOCUMENTS CONSIDERED TO BE RELEVANT**

| Category * | Citation of Document, with indication, where appropriate, of the relevant passages | Relevant to Claim No. |
|---|---|---|
| X, Y | JP, A, 55-118432 (Shionogi & Co., Ltd.) 11 September 1980 (11. 09. 80) Pages 1 to 6, upper part, page 13, lower part to page 14, (Family: none) | 1-11 |
| Y | JP, A, 49-48683 (Otsuka Pharmaceutical Co., Ltd.) 11 May 1974 (11. 05. 74) Pages 1 to 2 & FR, A, 2179715 & DE, A, 2302027 & GB, A, 1424571 | 1-5 |
| Y | JP, A, 51-34169 (Otsuka Pharmaceutical Co., Ltd.) 23 March 1976 (23. 03. 76) Page 1 (Family: none) | 1-5 |
| Y | JP, A, 49-94684 (Otsuka Pharmaceutical Co., Ltd.) 9 September 1974 (09. 09. 74) Page 1 (Family: none) | 1-5 |

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| October 1, 1987 (01. 10. 87) | October 19, 1987 (19. 10. 87) |
| **International Searching Authority** | **Signature of Authorized Officer** |
| Japanese Patent Office | |